# EUROPEAN PATENT APPLICATION

(11) **EP 4 108 179 A1**
(43) Date of publication of application: **28.12.2022**
(21) Application number: 21201927.7
(22) Date of filing: 11.10.2021
(51) Int. Cl.: A61B 8/08

(54) **ULTRASOUND SYSTEMS AND METHODS FOR MONITORING A VALSALVA MANOEUVRE**

(30) Priority: 22.06.2021 WO PCT/CN2021/101445
(71) Applicant: Koninklijke Philips N.V., 5656 AG Eindhoven (NL)
(72) Inventor: XU, Jinping, Eindhoven (NL); HONG, Xiaowei, Eindhoven (NL)
(74) Representative: Philips Intellectual Property & Standards

(57) **Abstract**

An ultrasound system for use in monitoring a Valsalva Manoeuvre, VM performed by a subject. The ultrasound system comprises a computing apparatus configured to: receive a measurement of abdominal pressure, P, for the subject whilst the subject is performing the VM, wherein the measurement of abdominal pressure was measured using a sensor external to the body for measuring abdominal pressure; and determine an indication of whether the subject has effectively performed the VM by comparing the measurement of abdominal pressure for the subject whilst the subject is performing the VM to a reference abdominal pressure, PRef, measured for the subject.

## Description

### FIELD OF THE INVENTION

Embodiments herein relate to ultrasound systems and methods for use in monitoring a Valsalva Manoeuvre (VM) performed by a subject. Some embodiments also relate to a consumer apparatus and an associated computing application for monitoring VMs performed by a subject.

### BACKGROUND OF THE INVENTION

Pelvic floor dysfunctions (PFDs) are major conditions that frequently occur in adult women, carrying a significant burden on the quality of life. The incidence of PDFs tends to increase with age of the population. PFDs can manifest with symptoms such as incontinence, constipation, and/or prolapsed pelvic organs. Pelvic floor weakness is frequently generalized and clinically underdiagnosed, and medical imaging evaluation is of major importance especially prior to surgical correction. Medical imaging, such as Pelvic floor Ultrasound (US) or trans-perineal US, is a widely accepted tool for diagnosing PDFs in clinical practice. Papers on the topic include the paper by Chamie, L.P, et.al, entitled: 'Translabial US and dynamic MR imaging of the pelvic floor: Normal anatomy and dysfunction', RadioGraphics, 2018, Vol. 38:287-308.; and AIUM&IUGA, 'AIUM/IUGA Practice Parameter for the Performance of Urogynecological Ultrasound Examinations', Journal of Ultrasound Medicine, 2019, Vol. 34:851-864.

The Valsalva manoeuvre (VM) is often used during US examinations to assess the state of a subject's (e.g. patient's) pelvic floor muscles. During a VM, a clinician asks the subject to push out her pelvic floor muscles as hard as she can (for example, for 10 to 16 seconds), as if the subject is bearing down to pass a stool or deliver a baby. When the clinician says to start the VM, the subject will need to start pushing as hard as she can and keep pushing as much as possible for the duration of the VM. When the clinician says to relax, the subject can stop pushing. It is important that the subject achieves her maximum "push" during the VM in order for effective examination. The Valsalva manoeuvre is described in more detail in the paper by Francisco J.O, et.al, entitled: 'The time factor in the assessment of prolapse and levator ballooning', Int Urogynecol J, 2012, Vol. 23: 175-178.

In one type of examination, the mobility of the pelvic organs during a Valsalva manoeuvre and shape and size of the Levator Hiatus (LH) are measured during routine examination and used to diagnose PFDs. The dimension of the LH at the maximal point of a VM (e.g. the point at which the pressure applied by the subject is maximal) is an important diagnostic index in pelvic floor ultrasound since it is widely used as a standardized parameter. For example: an area of the LH over 25 cm² is considered abnormal for western women, whilst an area greater than 22cm² is considered abnormal for Chinese women. Experienced ultrasound practitioners can achieve near 90% reliability at diagnosing PFDs by measuring the area of the LH during the maximal point of the VM (see paper by Rong Lu, et.al, 'Application of ultrasound in diagnosis of uterine prolapse by measuring area of levator hiatus', National Med Journal China, 2019, Vol. 99: 2315-2318).

### SUMMARY OF THE INVENTION

According to the study by Rong et al., cited above, some causes of inaccuracy in pelvic floor Ultrasound Examinations arise due to false negatives due to subjects not achieving their maximal Valsalva (e.g. not exerting their maximum possible pressure on the pelvic floor during the VM). In such cases the subject will not push the pelvic muscle out to its maximum extent and thus the maximal area of the LH will be underestimated. False negatives of this type can occur for a variety of reasons, for example, women may be reluctant to push to their maximum extent in a hospital setting, for example, due to the risk of urinary leakage.

Furthermore, some false negatives may arise due to the field of view (FOV) obtained during the imaging process not covering the pelvic floor muscles sufficiently to be able to reconstruct LH image. For example, if the FOV of the ultrasound probe is small or the probe is located in an unsuitable position, then the region of interest (ROI) for a 4D VM sequence will be reduced during dynamic VM and this can also lead to underestimation of the area of the LH (due to reconstructed C-plane from the selected one 3D volume image at maximal VM). These issues and others are addressed by embodiments herein.

Thus, according to a first aspect, there is provided an ultrasound system for use in monitoring a Valsalva Manoeuvre, VM performed by a subject. The ultrasound system comprises a computing apparatus configured to: receive a measurement of abdominal pressure, P, for the subject whilst the subject is performing the VM, wherein the measurement of abdominal pressure was measured using a sensor external to the body for measuring abdominal pressure; and determine an indication of whether the subject has effectively performed the VM by comparing the measurement of abdominal pressure for the subject whilst the subject is performing the VM to a reference abdominal pressure, PRef, measured for the subject.

According to a second aspect there is an apparatus for use in monitoring a Valsalva Manoeuvre, VM performed by a subject, the apparatus comprising: a sensor external to the body for measuring abdominal pressure; and a computing apparatus, wherein the computing apparatus is configured to: obtain a measurement of abdominal pressure, P, for the subject, from the sensor, whilst the subject is performing a VM; and send the obtained measurement of abdominal pressure, P, for the subject to an ultrasound system for use by the ultrasound system as a reference abdominal pressure, PRef, in determining whether the subject has effectively performed the VM. The apparatus of the second aspect may be a consumer apparatus accompanied by a computer application for use by the subject at home.

According to a third aspect there is a method performed by an ultrasound system for use in monitoring a Valsalva Manoeuvre, VM performed by a subject. The method comprises: receiving a measurement of abdominal pressure, P, for the subject whilst the subject is performing the VM, wherein the abdominal pressure was measured using a sensor external to the body for measuring abdominal pressure; and determining an indication of whether the subject has effectively performed the VM by comparing the measurement of abdominal pressure for the subject whilst the subject is performing the VM to a reference abdominal pressure, PRef, measured for the subject.

According to a fourth aspect there is a computer program product comprising a computer readable medium, the computer readable medium having computer readable code embodied therein, the computer readable code being configured such that, on execution by a suitable computer or processor, the computer or processor is caused to perform the method of the third aspect.

Thus according to embodiments herein, there is an apparatus, such as a consumer apparatus, for measuring a reference pressure exerted by a subject during a VM, and a complementary apparatus for receiving the reference pressure and using it to determine an indication of whether the subject has effectively performed the VM. For example, the reference pressure may be determined, using the apparatus of the second aspect, whilst the subject is at home, in familiar surroundings where they may practice their VMs. The pressure achieved during such practice may then be used in an Ultrasound examination to determine whether the maximum pressure achieved during the VM performed in the hospital setting is representative of the maximum VM pressure that the subject can achieve in the home setting, thus ensuring that the VM is effectively performed. Ensuring that the VM is correctly performed reduces false negative PDF diagnoses.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 shows a consumer apparatus comprising a belt and a computer application for use in measuring abdominal pressure of a subject during a VM according to some embodiment herein;
Fig. 2 shows an ultrasound imaging system according to some embodiments herein;
Fig. 3 shows a computing apparatus according to some embodiments herein;
Fig. 4 shows an example ultrasound system according to some embodiments herein;
Fig, 5 shows an example method according to some embodiments herein;
Fig. 6 shows an example method according to some embodiments herein;
Fig. 7 shows example ultrasound images obtained during an example ultrasound examination;
Fig. 8 shows example ultrasound images obtained during an example ultrasound examination; and
Fig. 9 shows example ultrasound images obtained during an example ultrasound examination.

### DETAILED DESCRIPTION OF EMBODIMENTS

As described briefly above, embodiments herein relate to systems, apparatus and methods therefor for monitoring a subject (e.g. patient) as the subject performs a Valsalva Manoeuvre (VM). The monitoring may be performed to determine whether the VM was performed correctly and/or to determine whether ultrasound (US) imaging data of the pelvic floor of the subject, obtained whilst the subject performed the VM, is likely to be suitable for assessing whether the subject has pelvic floor dysfunction (PDF).

The maximum extent of the LH during a Valsalva Manoeuvre is a standardised measurement used to diagnose whether the subject has PFD. As described above, one of the reasons that such examinations can lead to false negatives (e.g. where PFD is missed by the clinician) is if the subject does not push their pelvic floor muscles out to their maximum extent during the VM. If a maximum push is not achieved, then the maximum extent of the LH is not achieved either (e.g. the maximum extent of the LH that the subject should or can reach) and this leads to underestimation of the LH area (which is reference standard for diagnosing PFD).

Embodiments herein propose a "training system" whereby the subject is given an apparatus with which to monitor themselves as they perform VMs e.g. at home. The training system records the abdominal pressure measurements in the home as reference pressures for the subject. The reference pressures obtained in the home setting may be used as a bench mark in the hospital setting to determine whether the subject is pushing to their maximum extent possible during a hospital-monitored VM. If the subject does not achieve the reference pressure (or a pressure close enough to the reference pressure) in the hospital setting then they may be asked to repeat the VM, for example.

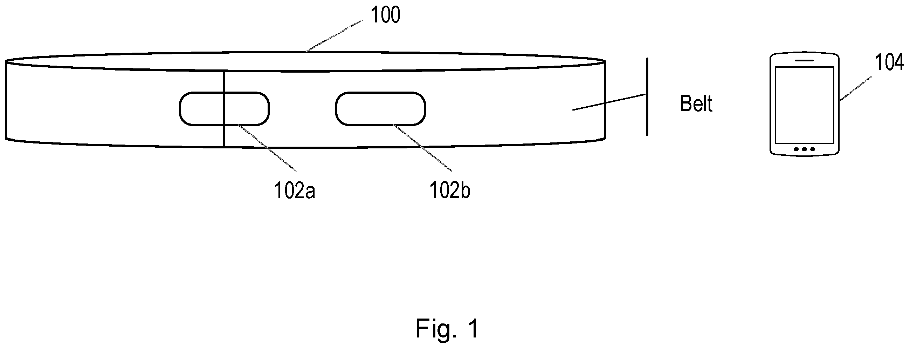

An example apparatus is illustrated in Fig. 1 which shows an apparatus for use in monitoring a Valsalva Manoeuvre, VM performed by a subject. In Fig. 1 the apparatus is a belt 100 comprising one or more external pressure sensors 102a, 102b. The belt is worn around the abdomen of the subject and holds the pressure sensor(s) against the subject's body. The apparatus 100 further comprises a computing apparatus 104 configured to obtain a measurement of abdominal pressure, P, for the subject, from the sensor, whilst the subject is performing a VM. The belt may be used to monitor a plurality of (practice) VMs performed by the subject, e.g. in order to determine the maximum pressure that the subject can achieve in their own home/under relaxed conditions. The historical or practice VMs may be performed at the same location (e.g. in the home) for comparison purposes between different pressure measurements, but performed by the same subject. The computing apparatus 104 is then configured to send an obtained measurement of abdominal pressure, P, for the subject to an ultrasound system for use by the ultrasound system as a reference abdominal pressure (e.g. a pressure that the subject is known to be able to achieve), PRef, for use in determining whether the subject has effectively performed a VM.

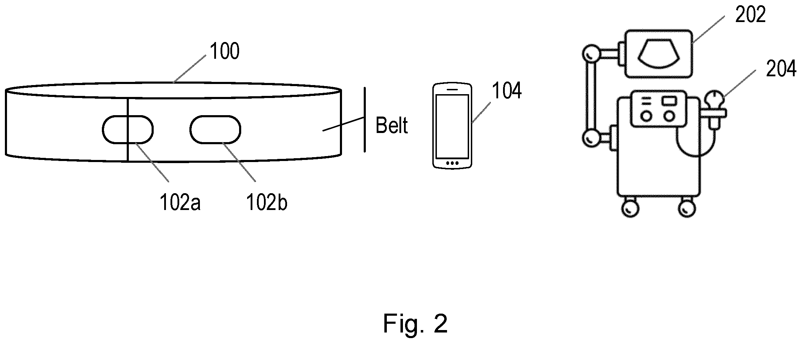

When the subject subsequently goes for an US pelvic floor examination, e.g. in a hospital setting, an US system such as that illustrated in Fig. 2 may be used. In the hospital setting, a belt similar to that described with respect to Fig. 1, may be used to measure the abdominal pressure of the subject whilst the subject performs the VM. The belt will measure the abdominal pressure, and a computing apparatus in the ultrasound system 202 is configured to receive the measurement of abdominal pressure, P, and determine an indication of whether the subject has effectively performed the VM by comparing the measurement of abdominal pressure for the subject whilst the subject is performing the VM to the reference abdominal pressure, PRef, measured for the subject (e.g. as obtained using the consumer apparatus 100 in the home setting). In this way, an indication can be given to the clinician of whether the subject has correctly performed the VM.

In this way, the apparatus in Fig. 1 can be used to monitor the subject as they perform a VM at home, before examination. The ultrasound system 202, 204 in Fig. 2 can then be used to providing guidance tool to an ultrasound practitioner to help identify if a VM performed during an ultrasound examination is properly performed for a specific task before the subject leaves the examination room.

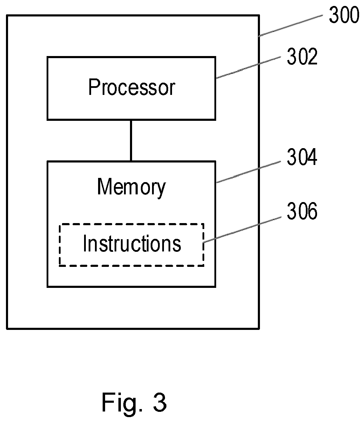

In more detail, and turning now to Fig. 3, some embodiments herein are performed using a computing apparatus such as the computing apparatus 300. Generally, the computing apparatus 300 may form part of a computer or system e.g. such as a mobile phone, tablet, laptop, desktop computer or other computing device. In some embodiments, the apparatus 300 may form part of a distributed computing arrangement or the cloud.

The computing apparatus comprises a memory 304 comprising instruction data representing a set of instructions and a processor 302 (e.g. processing circuitry or logic) configured to communicate with the memory and to execute the set of instructions. Generally, the set of instructions, when executed by the processor, may cause the processor to perform any of the embodiments of the method 400 or the method 500 as described below.

The processor 302 can comprise one or more processors, processing units, multi-core processors or modules that are configured or programmed to control the computing apparatus 300 in the manner described herein. In particular implementations, the processor 302 can comprise a plurality of software and/or hardware modules that are each configured to perform, or are for performing, individual or multiple steps of the method described herein. The processor 302 can comprise one or more processors, processing units, multi-core processors and/or modules that are configured or programmed to control the computing apparatus 300 in the manner described herein. In some implementations, for example, the processor 302 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the methods described herein.

The memory 304 is configured to store program code that can be executed by the processor 302 to perform the method described herein. Alternatively or in addition, one or more memories 304 may be external to (i.e. separate to or remote from) the computing apparatus 300. For example, one or more memories 304 may be part of another device. Memory 304 can be used to store any information or data received, calculated or determined by the processor 302 of the computing apparatus 300 or from any interfaces, memories or devices that are external to the computing apparatus 300. The processor 302 may be configured to control the memory 304 to store such information.

In some embodiments, the memory 304 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 3 only shows the components required to illustrate this aspect of the disclosure and, in a practical implementation, a computing apparatus 300 may comprise additional components to those shown. For example, a computing apparatus 300 may further comprise a display. A display may comprise, for example, a computer screen, and/or a screen on a mobile phone or tablet. A computing apparatus may further comprise a user input device, such as a keyboard, mouse or other input device that enables a user to interact with the computing apparatus, for example, to provide initial input parameters to be used in the method described herein. A computing apparatus 300 may comprise a battery or other power supply for powering the computing apparatus 300 or means for connecting the computing apparatus 300 to a mains power supply.

In some embodiments, a computing apparatus 300 may be comprised in (e.g. form part of) an ultrasound system. For example, an ultrasound system may comprise the computing apparatus 300 described above, and further comprise a transducer with which to record the sequence of ultrasound image data.

In some embodiments herein, a computing apparatus 300 is comprised in (e.g. forms part of) a consumer apparatus 104 as illustrated in Fig. 1.

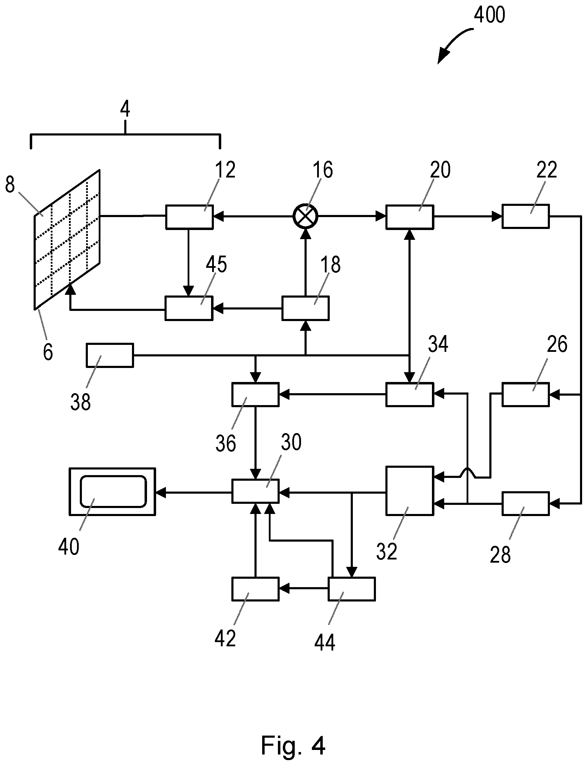

Turning now to other embodiments, some embodiments herein relate to ultrasound systems. Fig. 4 shows an example ultrasound system 400. The ultrasound system 400 may comprise a computer apparatus 300 as described above. In other embodiments, components of the ultrasound system 400 may be adapted to perform the method 400 as described below.

The system 400 comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 may be coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a microbeamformer 12, the transducer array may be operated directly by a main system beamformer (not shown in Fig. 4).

The system 400 may further comprise a transmit/receive (T/R) switch 16, which the microbeamformer 12 can be coupled to and which switches the array between transmission and reception modes. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

It is noted that in an alternative embodiment, where instead of a microbeamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one-dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 4 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example,: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38, such as the seed points for locating the surface of the muscle, as described in detail below.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The skilled person will appreciate that the detail provided above and the components illustrated in Fig. 4 are an example only and that an ultrasound system may have different components to those illustrated therein.

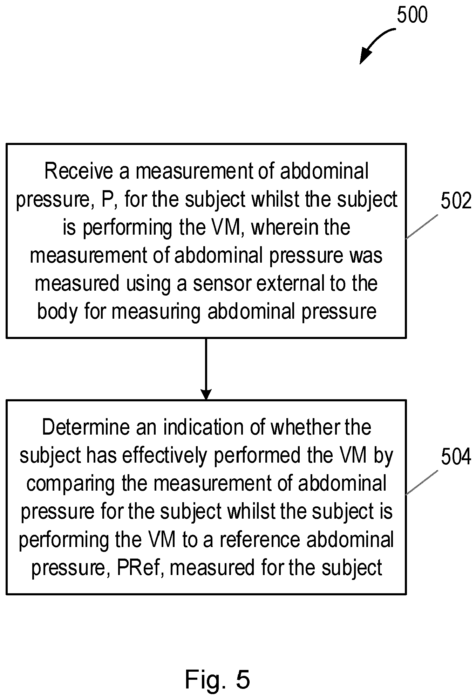

Turning to Fig. 5, there is a computer implemented method 500 performed by an ultrasound system for use in monitoring a Valsalva Manoeuvre, VM performed by a subject. Embodiments of the method 500 may be performed, for example by an ultrasound system such as the ultrasound systems 202, 204 or 400 described above. In some embodiments the method 500 may be performed by a computing apparatus 300 described above.

Briefly, in a first step 502, the method 500 comprises receiving a measurement of abdominal pressure, P, for the subject whilst the subject is performing the VM, wherein the measurement of abdominal pressure was measured using a sensor external to the body for measuring abdominal pressure. In a second step 504, the method 500 comprises determining an indication of whether the subject has effectively performed the VM by comparing the measurement of abdominal pressure for the subject whilst the subject is performing the VM to a reference abdominal pressure, PRef, measured for the subject.

In more detail, the measurement of abdominal pressure received in step 502 is measured using a sensor external to the body for measuring abdominal pressure. In some embodiments, the sensor is comprised in a belt that is worn around the subject's abdomen, as illustrated in Figs 1 and 2 above. In some embodiments, the sensor measures the pressure in a direction perpendicular to the abdominal muscles during the VM (e.g. outward pressure on the belt).

In other embodiments, the sensor may be embedded in a patch that is attached or stuck to the subject, or measured using a handheld sensor. The skilled person will appreciate that these are merely examples and that any external sensors suitable for measuring abdominal pressure may be used.

In step 504 the method 500 then comprises determining an indication of whether the subject has effectively (e.g. correctly; exerting a pressure close to the maximum possible pressure achievable for the subject) performed the VM by comparing the measurement of abdominal pressure for the subject whilst the subject is performing the VM to a reference abdominal pressure, PRef, measured for the subject.

Generally, the reference pressure is a previous pressure achieved by the subject, or a target pressure for the subject. The reference pressure may be based on a pressure that the subject has previously achieved whilst performing a VM.

The reference pressure may be a maximum historical abdominal pressure measurement measured for the subject, determined from a plurality of historical abdominal pressure measurements, each of the plurality of historical abdominal pressure measurements having been taken as the subject performed a VM.

As an example, the plurality of historical abdominal pressure measurements may have been made using a consumer apparatus as described above with respect to Fig. 1. The plurality of historical abdominal pressure measurements may have been made whilst the subject was at home, or in some other environment where the subject is able to be comfortable. The reference pressure may be the maximum pressure previously obtained by the subject during a VM, for example, the maximum value of the plurality of historical abdominal pressure measurements.

In step 504, the measurement of abdominal pressure for the subject may be compared to the reference pressure by taking a ratio of P/PRef. In other words, the indication of whether the subject has effectively performed the VM may be based on a measure of P/Pref.

For example, the indication may be a value of P/PRef. In other examples, the indication may indicate whether P/PRef is greater a first predefined threshold. The first predefined threshold may be, for example, about 0.9. For example, the indication may indicate if the subject achieves a pressure 90% or more of the maximum recorded historical pressure for the subject. As another example, if the subject achieves a pressure 90% or more of the maximum recorded historical pressure for the subject, then the indication may indicate that the VM was performed correctly.

In some embodiments, the method may comprise sending a signal to a display to cause the display to provide an indication of whether P/PRef is greater than the first predefined threshold. As such, the indication may be displayed on the display for use by a clinician in determining whether the subject has correctly (or sufficiently) performed a VM. From this, the clinician can use the indication to determine whether US images obtained during the VM were taken at an abdominal pressure close enough to the subject's maximal pressure, and thus whether the US images are suitable for diagnostic purposes e.g. suitable for use in diagnosing whether the subject has PFD.

In some embodiments, the method may further comprise obtaining ultrasound data of the pelvic floor of the subject whilst the subject is performing the VM. The ultrasound image data may be obtained as part of a pelvic floor examination. The ultrasound images may for example, be Pelvic floor US images, trans-perineal US images or translabial ultrasound images.

The ultrasound data may comprise raw data or radiofrequency (RF) signal data. The US data may alternatively or additionally be formatted into image data in a format such as e.g. the Digital Imaging and Communications in Medicine (DICOM) image format or any other suitable image format. The US data may be three-dimensional or two dimensional. Generally a sequence of images may be obtained. For example, at 1 second intervals throughout performance of the VM.

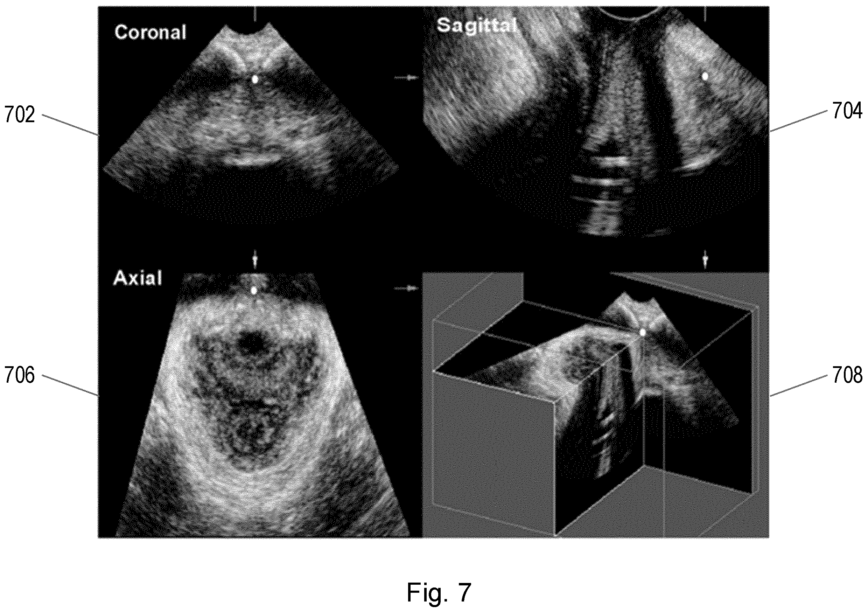

Fig. 7 illustrates an introital static 3D volume processed to demonstrate the relationship of the three orthogonal planes-coronal 702, sagittal 704, and axial 706 in the context of a pelvic floor ultrasound examination. The cursor dot seen in each plane represents the exact same spot in each plane.

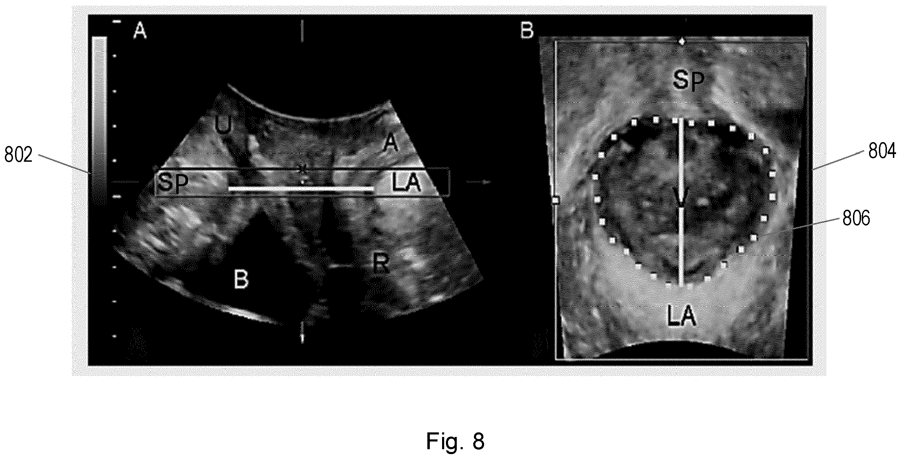

Generally, the method 500 may comprise making measurements of the area of the levator hiatus, LH which is illustrated in Fig. 8. Fig. 8 shows a mid sagittal 2D image at A-plane from a four-dimensional US acquisition for organ descent and hiatal ballooning or over-distensibility (85 degrees acquisition angle). The region of interest (box in A in Fig. 802) is set between the symphysis pubis (S) on the left and levator ani (LA) on the right. A indicates anal canal; B bladder; R, rectal ampulla; U, urethra; and V, vagina. The dotted contour 806 in the right-hand image 804 is the circumference or perimeter of the Levator hiatus (LH) in the plane of minimal dimensions; the solid white line in both 802 and 804 is the hiatal anteroposterior (AP) diameter in the midsagittal (anteroposterior) plane.

Generally, the midsagittal plane is determined from the 3D volume frame in Fig. 7 as shown in Fig. 8, and C-plane reconstruction/rending for the LH image is implemented by selection of key points and drawing a line through the key points as minimal dimension plane.

The area of the LH may be determined, for example, by segmenting the US image data to determine the boundaries of the LH and determining the area therein. The skilled person will be familiar with segmentation and methods for performing segmentation of an image, but briefly, image segmentation involves extracting shape/form information about the objects or shapes captured in an image. This may be achieved by converting the image into constituent blocks or "segments", the pixels or voxels in each segment having a common attribute.

One method of image segmentation is Model-Based Segmentation (MBS), whereby a triangulated mesh of a target structure (such as, a mesh of the LH) is adapted in an iterative fashion to features in an image. Model-based segmentation has been used in various applications to segment one or multiple target organs from medical images, see for example, the paper by Ecabert, O., et al. 2008 entitled "Automatic Model-Based Segmentation of the Heart in CT Images"; IEEE Trans. Med. Imaging 27 (9), 1189―1201. Another segmentation method uses machine learning (ML) models to convert an image into a plurality of constituent shapes (e.g. block shapes or block volumes), based on similar pixel/voxel values and image gradients.

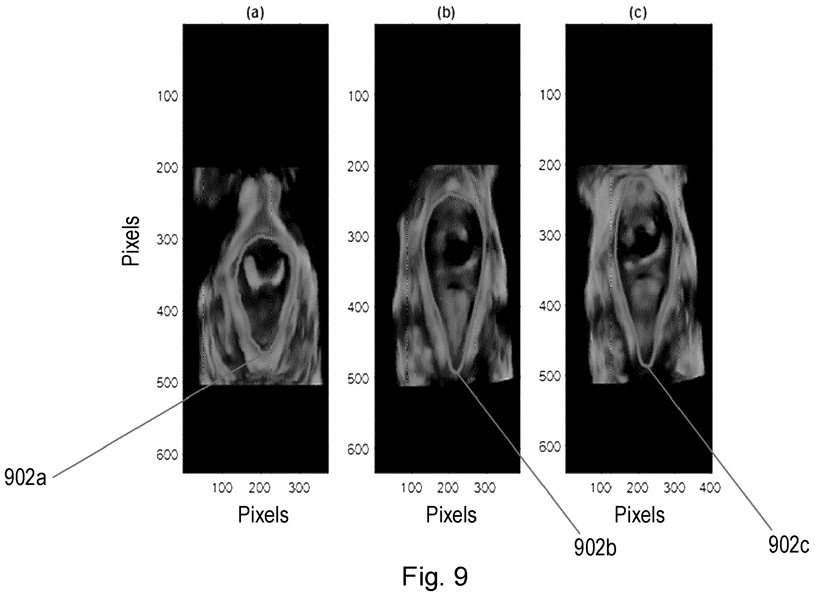

Fig. 9 shows segmented images showing the LH for a subject at three different time points in a VM. The LH in each image is indicated by the lines 902a, 902b and 902c respectively. Fig. 9(a) indicates the LH at rest (baseline measurement). The area of the LH and AP diameter in each image is (a) :12.9 cm^2/4.7cm; (b): 20.5 cm^2/6.9cm and (c):21.9 cm^2/7.62cm. In this example, the change in AP diameter compared to the baseline is (b): (6.9-4.7)/4.7 = 46.81% and (c): (7.62-4.7)/4.7=62.13%. The change in Area compared to the baseline is (b): (20.5-12.9)/12.9 = 58.92% and (c) (21.9-12.9)/12.9 = 69.77.

Turning back to Fig. 5, the method 500 may comprise making measurements of the area of the levator hiatus, LH, of the subject in the obtained ultrasound data at a plurality of time points as the subject performs the VM (e.g. measuring the LH in each of a sequence of US images as the VM progresses). The plurality of time points may be at one predefined interval (for example: at 6 second intervals throughout the VM). The skilled person will appreciate however that 6 seconds is merely an example and that the plurality of time points could be taken at consecutive time intervals of any length. Another simple approach is to select 3 representative LH images as first one for the very beginning of the VM, second one located at the middle of the VM, the last one located at either maximal VM time point or closer to the end of the VM.

It has been observed that if a VM is performed correctly, then the LH expands in a predictable manner, e.g. according to a profile. Thus, the shape-profile of the LH during a VM can be analysed to determine whether the VM was performed effectively. Thus, in some embodiments, the method 500 may further comprise determining the indication based on a comparison of the measurements of the area of the LH at the plurality of time points with a predefined profile of area measurements of the LH during a VM.

Generally, the predefined profiles may be obtained empirically based on measured profiles of different test subjects. Example predefined profiles that may be used are found in the paper by: 'Levator co-activation is a significant confounder of pelvic organ descent on Valsalva maneuver', Ultrasound Obstet Gynecol, 2007, Vol. 30: 346-350.

In some embodiments, the International Continence Society Prolapse Quantification System (ICS POP-Q) assessment profiles may be used. This system has 5 standardized curves for 5 different conditions as following:
1) Normal subject: small LHA change at maximum VM pressure (MVM) compared to at rest;
2) Stage 1 according to International Continence Society Prolapse Quantification System (ICS POP-Q) assessment
3) ICS-POP-Q stage 2
4) ICS-POP-Q stage 3
5) ICS-POP-Q stage 4.

In one embodiment, the measurements of the area of the LH at the plurality of time points can be compared with a predefined profile of area measurements as follows:
The US volume sequence number at which the LH area is maximal, LHMax (e.g. for the maximal VM pressure) may be determined by the maximal pelvic organ decent.
For the determined US volume, the area of the LH (LHA) is then determined for every preceding US image in the sequence, so that a sequence of area measurements, for example, LHA(1:10) (note that 10 measurements is merely an example and that generally, the sequence of area measurements may comprise any number of measurements) are obtained (e.g LHA(0) being at rest, and corresponding to LHRest, up to LHA(10) corresponding to LHAMax).
Compare the obtained LHA curve with standardized curve, and if the difference between them is less than a predefined profile difference, then the indication in step 504 may indicate that the subject has effectively performed the VM, otherwise; if the difference is greater than the predefined profile difference then the indication in step 504 may indicate that the subject has not effectively performed the VM.

Other image features can also be used to determine whether the VM was performed effectively. For example, in some embodiments, the method 500 may further comprise determining from the measurements of the area of the LH, a maximum extent of the LH, LHAMax during the VM and an extent of the LH at rest, LHArest. The indication in step 504 may then further indicate whether a percentage change between LHAMax and LHArest is greater than a threshold percentage change. For example, if (LHAMax -LHArest)/LHArest is less than a pre-determined threshold (for example: 10%) then this VM may be ruled out and the indication may indicate that the VM was not performed correctly.

Relative change in levator hiatal dimensions (for example: LH area) may also be used to determine whether the VM has been performed effectively. For example, the relative change between between rest and the maximum point (e.g. labelled as index0 at range from 0% to 70% an example for maximal change in this case) is (LHAMax -LHArest)/LHArest. Index series of relative changes (for example: index1, index2, index3, et.al) at several pressure measurement points can also be computed along the pressure waveform at different time markers. From such index series, a maximal change in measurements can be determined and this can also be used to determine whether the VM is effective or not.

For example, the method 500 may further comprise determining from the ultrasound data a maximal change in measurements of the area of the LH with respect to time. The indication in step 504 may then further indicate whether the maximal change in measurements of the area of the LH with respect to time is greater than a second predefined threshold. In some embodiments, if the slope is larger than the second predefined threshold and also the abdominal pressure is suitable (e.g. P/Pref > the first predefined threshold) then the VM is considered to have been effectively performed.

In some embodiments, thehiatal anteroposterior (AP) diameter can also be used to determine whether the VM has been performed effectively. For example, step 504 may further comprise determining from the ultrasound data a hiatal AP diameter during the VM, and determining from the ultrasound data an anteroposterior diameter at rest. In such embodiments, the indication may further indicate whether the hiatal anteroposterior diameter during the VM is larger than the anteroposterior diameter at rest. This is based on the fact that during an effective/correct VM, the hiatal anteroposterior diameter should be larger than the anteroposterior diameter at rest. As such, this can be indicated to the clinician.

In some embodiments, the bladder neck descent can be used to determine whether the VM has been performed correctly. For example, the bladder neck is expected to descend during correct performance of a VM. As such, in some embodiments, the step 504 may further comprise determining from the ultrasound data a measure of bladder neck descent (BND) for the subject during the VM. In such embodiments, the indication may further indicate whether the BND is greater than a predefined threshold BND value.

An appropriate level for the predefined threshold BND value may be determined, for example, empirically from population studies. As another example, the threshold BND value may be set at the symphysis pubis (SP), such that if level of the bladder neck is over the SP, then this is an indication that the VM is effective.

In an example embodiment, in step 504, the following sequence of rules may be used to determine if a VM is effective:
1) If index0 is smaller than a predefined threshold percentage change (for example: 10%), then rule out this examination (e.g. provide an indication that the VM was not performed effectively);
2) If index0 is bigger than the predefined threshold percentage change, then the VM passes the first step checking as candidate pool. Using index series (for example: index1, index2, index3, et.al) to determine the maximal slope the series;
3) If the maximal slope is smaller than a second predefined threshold (as described above), then consider this VM examination as not effective; otherwise if the slope is larger than the second predefined threshold and also the abdominal pressure is suitable (e.g. P/Pref > first predefined threshold), then consider this VM examination as effective.
4) If the hiatal diameter anteroposterior diameter: APD) at VM is smaller than APD at rest, this indicates the VM is not effective; otherwise if the APD at VM is much bigger than APD at the rest (for example: 10%), then the VM is effective;
5) Check if the bladder neck descent (BND in mm) gets larger during a few VMs after biofeedback training, this indicates the subject will be more effective at VM until the value of BND is close to its maximum for the subject, the bigger BND is, the more effective VM is (for example: if the level of the bladder neck is over the SP). As noted above, the optimal threshold for BND could be obtained from population study for many subjects.

Turning back to the method 500, in some embodiments, the method may further comprise instructing a display to display the indication on the display for use by a physician in determining whether the obtained ultrasound data is suitable for use in determining whether the subject has pelvic floor dysfunction. Generally, the display may display the data and/or the derived values of any of the indications described above. In some embodiments the indication may further provide advice to the clinician, for example: "The maximum abdominal pressure during the VM is less than 90% of the Reference Pressure recorded for the subject" or "The maximum abdominal pressure during the VM is less than 90% of the Reference Pressure recorded for the subject, the VM needs to be repeated". The indication may indicate that the obtained images are unlikely to be suitable for diagnostic purposes, for example, the indication may provide a warning that if the VM is not performed effectively then there is a risk that a PDF will be missed. The skilled person will appreciate that these are merely examples however and that the indication may be presented on a display in a wide range of different ways.

Thus, using the metrics above, an indication can be provided to a clinician performing a pelvic floor examination, that can be used by the clinician to determine whether the subject has correctly performed a VM, and thus whether the US images obtained are suitable for use in diagnosing PDFs.

Turning now to other embodiments, in one example system, there is an ultrasound system comprising a plurality of computing modules for analysing US image data obtained as a subject performs a VM that comprises some of the elements described above. For example, the computing modules may comprise:

A first module to compute the area of the LH at contraction situation from pelvic floor ultrasound image.

A second module to compute the area of the LH at the beginning of the VM and at its maximum condition (e.g. maximum pressure).

A user interface to confirm if the LH boundary is well detected.

A third module to obtain the reference abdominal pressure for the subject PRef (e.g. as obtained at home using an apparatus such as the apparatus 100 described above) and the abdominal pressure P obtained in the examination room as the subject performs a VM during an ultrasound examination

A fourth module for determining an indication of whether the VM has been performed correctly. This could determine any of the types of indication described above, for example, a measure of P/Pref and/or one or more of the LH area measurements described above.

A fifth module that, for a given 2D/3D pelvic floor ultrasound image, provides information on the representative 2D/3D image and corresponding shapes for the detected LH (e.g. obtained via segmentation as described above) with useful LH dimensions.

The skilled person will appreciate that this is merely an example illustrating how some of the teachings described herein might be arranged in an ultrasound system, and that other combinations of modules are also possible.

Turning now to other embodiments, as described above with respect to Fig. 1, in embodiments herein, the reference abdominal pressure, Pref, is determined for the subject using a sensor external to the body. For example, the sensor may be held against the body by means of a belt 100 worn around the waist, or a patch.

More generally, in some embodiments, there is an apparatus 100 for use in monitoring a Valsalva Manoeuvre, VM performed by a subject. The apparatus 100 comprises a sensor external to the body for measuring abdominal pressure; and a computing apparatus such as the computing apparatus 300 described above.

As described above, the sensor may be incorporated into a consumer device. For example, the sensor may be comprised in a belt worn around the subject's abdomen or waist. The sensor may be any type of pressure sensor that can measure force perpendicular to the skin surface. The belt may comprise one or more such pressure sensors and/or sensors for measuring stretch of the belt, e.g. pull/expansion force sensors.

In other embodiments, the pressure sensor may be embedded in a patch stuck to the skin, or a handheld device e.g. on the surface of the stomach against which the subject pushes against.

The computing apparatus may be comprised in (e.g. form part of) a computing device such as a mobile phone, tablet computer, laptop, smart watch, or any other computing device configured to perform the functionality described herein. In some embodiments, the computing apparatus runs a computing application, "app".

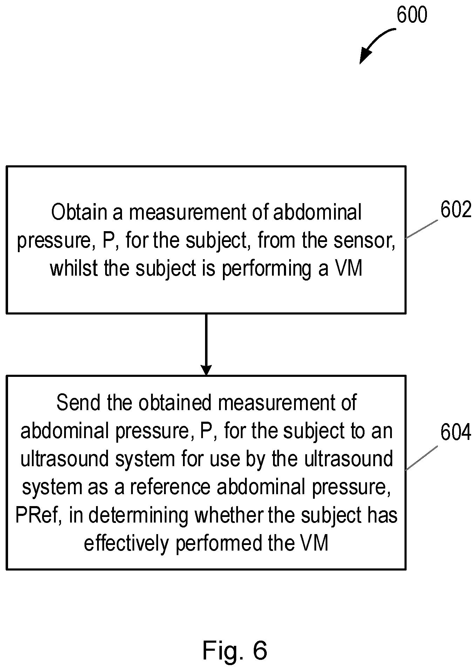

The computing apparatus is configured to perform the method 600 illustrated in Fig. 6. In brief, the computing apparatus is configured to: obtain 602 a measurement of abdominal pressure, P, for the subject, from the sensor, whilst the subject is performing a VM. The computing apparatus is further configured to send 604 the obtained measurement of abdominal pressure, P, for the subject to an ultrasound system for use by the ultrasound system as a reference abdominal pressure, PRef,,in determining whether the subject has effectively performed the VM.

In some embodiments, the method 600 is performed by an app (e.g. on a smartphone, smart watch or similar) that has user interface to guide a subject step by step to remind the subject to perform VM training periodically. This may make the subject more comfortable performing the VM as the subject can practise at home, and may thus provide a more accurate indication of the (true) maximum pressure (PRef) that the subject can achieve during a VM.

The computing apparatus may be configured to give feedback when a subject performs a VM. For example, the computing apparatus may:
- ask the subject to attach the external sensor at a suitable location at which they feel comfortable;
- obtain force measured by pressure and/or stretch sensors around abdomen during (a) contraction condition and (b) Valsalva process (from beginning point to its maximum state). As noted above and shown in Fig. 1, the device used for this purpose can be a belt with embedded pressure sensor and/or pull/expansion force sensor.

If the subject is doing the expected maximum Valsalva, then the detected pressure is considered as the maximum pressure value. After a period of time training (for example: a week), if the subject has improved their Valsalva capability (e.g. increased their maximal pressure), then a new high maximum pressure value may be set as the target for the subject to achieve in the next week. After 3~4 weeks training, if the maximum pressure value is stable, that that value will be considered as the reference pressure PRef, reflective of the maximal pressure that the subject can obtain. This value of PRef will then be sent (in step 604) be sent for use by the ultrasound system (e.g. for use in the method 500 during an ultrasound examination, as described above).

Generally, the apparatus 100 may record pressure measurements as the subject performs practice VMs. For example, the apparatus 100 may record the maximum pressure measurement of a plurality of VMs performed by the subject. The maximum of the plurality of measurements may then be sent to the US system (e.g. to a hospital/doctors surgery or hospital facility) for use by an US system as the reference pressure, PRef in the method 500 as described above.

In this way, the apparatus 100 may be used to monitor and record the maximum abdominal pressure achieved by the subject during the VM so as to produce an individualised target pressure PRef that the subject should achieve during the hospital setting, if the VM is performed to the subjects maximal pushing ability. Through the apparatus 100, a subject can be trained to correctly perform the VM through coaching.

In summary, the apparatus described herein can be used to determine the maximal pressure PRef that a subject is capable of exerting on their pelvic floor and this can then be used, in real-time, in an ultrasound system to determine whether US imaging data collected as a subject performs a VM is suitable for use in a subsequent diagnostic process, e.g. suitable for analysing whether the subject has PFD.

Turning now to other embodiments, in another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An ultrasound system for use in monitoring a Valsalva Manoeuvre, VM performed by a subject, the ultrasound system comprising a computing apparatus configured to:
receive a measurement of abdominal pressure, P, for the subject whilst the subject is performing the VM, wherein the measurement of abdominal pressure was measured using a sensor external to the body for measuring abdominal pressure; and
determine an indication of whether the subject has effectively performed the VM by comparing the measurement of abdominal pressure for the subject whilst the subject is performing the VM to a reference abdominal pressure, PRef, measured for the subject.

2. . An ultrasound system as in claim 1 wherein the reference abdominal pressure is a maximum historical abdominal pressure measurement measured for the subject, determined from a plurality of historical abdominal pressure measurements, wherein each of the plurality of historical abdominal pressure measurements were taken as the subject performed a VM.

3. . An ultrasound system as in claim 1 or 2 wherein the sensor is comprised in a belt that is worn around the subject's abdomen and/or wherein the sensor measures the pressure in a direction perpendicular to the abdominal muscles during the VM

4. . An ultrasound system as in any one of the preceding claims wherein the indication is based on P/PRef and wherein the computing apparatus is further configured to:
send a signal to a display to cause the display to provide an indication of whether P/PRef is greater than a first predefined threshold.

5. . An ultrasound system as in any one of the preceding claims wherein the computing apparatus is further configured to:
obtain ultrasound data of the pelvic floor of the subject whilst the subject is performing the VM; and
make measurements of the area of the levator hiatus, LH, of the subject in the obtained ultrasound data at a plurality of time points as the subject performs the VM.

6. . An ultrasound system as in claim 5 wherein the computing apparatus is further configured to:
determine the indication based on a comparison of the measurements of the area at the plurality of time points with a predefined profile of area measurements of the LH during a VM.

7. . An ultrasound system as in claim 6 wherein the computing apparatus is further configured to:
determine from the measurements of the area of the LH, a maximum extent of the LH, LHAMax during the VM and an extent of the LH at rest, LHArest; and wherein
the indication further indicates whether a percentage change between LHAMax and LHArest is greater than a threshold percentage change.

8. . An ultrasound system as in claim 5, 6, or 7 wherein the computing apparatus is further configured to:
determine from the ultrasound data a maximal change in measurements of the area of the LH with respect to time; and
wherein the indication further indicates whether the maximal change in measurements of the area of the LH with respect to time is greater than a second predefined threshold.

9. . An ultrasound system as in claim 5, 6, 7 or 8 wherein the computing apparatus is further configured to:
determine from the ultrasound data a hiatal anteroposterior (AP) diameter during the VM;
determine from the ultrasound data an AP diameter at rest; and
wherein the indication further indicates whether the hiatal anteroposterior diameter during the VM is larger than the anteroposterior diameter at rest.

10. . An ultrasound system as in claim 5, 6, 7, 8, or 9 wherein the computing apparatus is further configured to:
determine from the ultrasound data a measure of bladder neck descent, BND, for the subject during the VM; and
wherein the indication further indicates whether the BND is greater than a predefined threshold BND value.

11. . An ultrasound system as in any one of claims 5 to 10 wherein the computing apparatus is further configured to instruct a display to display the indication on the display for use by a physician in determining whether the obtained ultrasound data is suitable for use in determining whether the subject has pelvic floor dysfunction.

12. . An apparatus for use in monitoring a Valsalva Manoeuvre, VM performed by a subject, the apparatus comprising:
a sensor external to the body for measuring abdominal pressure; and
a computing apparatus, wherein the computing apparatus is configured to:
obtain a measurement of abdominal pressure, P, for the subject, from the sensor, whilst the subject is performing a VM; and
send the obtained measurement of abdominal pressure, P, for the subject to an ultrasound system for use by the ultrasound system as a reference abdominal pressure, PRef, in determining whether the subject has effectively performed the VM.

13. . An apparatus as in claim 12, wherein the sensor is comprised in a belt that is to be worn around the subject's abdomen and/or wherein the sensor measures the pressure in a direction perpendicular to the abdominal muscles during the VM.

14. . A method performed by an ultrasound system for use in monitoring a Valsalva Manoeuvre, VM performed by a subject, the method comprising:
receiving a measurement of abdominal pressure, P, for the subject whilst the subject is performing the VM, wherein the abdominal pressure was measured using a sensor external to the body for measuring abdominal pressure; and
determining an indication of whether the subject has effectively performed the VM by comparing the measurement of abdominal pressure for the subject whilst the subject is performing the VM to a reference abdominal pressure, PRef, measured for the subject.

15. . A computer program product comprising a computer readable medium, the computer readable medium having computer readable code embodied therein, the computer readable code being configured such that, on execution by a suitable computer or processor, the computer or processor is caused to perform the method as claimed in claim 14.
